# EUROPÄISCHE PATENTSCHRIFT

(11) **EP 3 423 148 B1**
(45) Veröffentlichungstag und Bekanntmachung des Hinweises auf die Patenterteilung: **02.09.2020**
(21) Anmeldenummer: 17707519.9
(22) Anmeldetag: 23.02.2017
(51) Int. Cl.: A61N 1/05

(54) **IMPLANTIERBARE MANSCHETTENELEKTRODE**
IMPLANTABLE CUFF ELECTRODE
ÉLECTRODE À GAINE IMPLANTABLE

(30) Priorität: 29.02.2016 DE 102016103597
(43) Veröffentlichungstag der Anmeldung: 09.01.2019
(73) Patentinhaber: CorTec GmbH, 79108 Freiburg (DE)
(72) Erfinder: SCHÜTTLER, Martin, 79312 Emmendingen (DE); ORDONEZ, Juan Sebastian, 9000 Gent (BE); RICKERT, Jörn, 79104 Freiburg (DE); STIEGLITZ, Thomas, 79110 Freiburg (DE)
(74) Vertreter: 2s-ip Schramm Schneider Bertagnoll Patent- und Rechtsanwälte Part mbB
(86) Internationale Anmeldenummer: PCT/EP2017/054259
(87) Internationale Veröffentlichungsnummer: WO 2017/148806

(56) Entgegenhaltungen:
- US-A- 5 487 756
- US-A1- 2006 116 739
- US-A1- 2010 298 916
- US-A1- 2013 261 721

## Beschreibung

Die vorliegende Erfindung betrifft eine in den Menschlichen oder tierischen Körper implantierbare Manschettenelektrode und ein Verfahren zu deren Herstellung.

In der Medizintechnik werden implantierbare Systeme verwendet, um mit dem Nervensystem zu interagieren und ausgefallene Körperfunktionen zu unterstützen oder wieder herzustellen, ebenso wie Funktionsstörungen von Organen zu beheben. Dafür werden Manschettenelektroden benötigt und um die Nerven platziert. Auf der Innenseite tragen die Manschetten elektrische Kontakte, die das Aufzeichnen elektrischer Aktivität ermöglichen und/oder eine elektrische Beeinflussung der natürlichen Nervenaktivität erlauben. Anforderungen an eine Manschettenelektrode sind u.a.:
Die elektrischen Kontakte sollen sich in unmittelbarer Nähe zum Zielnerv befinden, um eine gute elektrische Kopplung zum Nervengewebe zu bieten.

Die Manschette muss eine sehr gute elektrische Isolation über ihre gesamte Länge gegenüber der Umgebung sicherstellen, um zu verhindern, dass während des Aufzeichnens von Nervensignalen elektrische Signale z.B. von benachbartem Muskelgewebe in die Aufzeichnung einkoppeln.

Die Manschette sollte aus sehr weichem Material bestehen, damit unnötige mechanische Irritation und/oder Beeinträchtigung des Nerven, z.B. durch harte oder gar scharfe Kanten weitgehend vermieden wird.

Die Manschette sollte erlauben, dass der Nerv, z.B. als Reaktion auf die Implantationsprozedur vorübergehend anschwillt; denn schwillt ein Nerv in einer Manschette mit unveränderlichem Durchmesser an, können die im Nerv befindlichen Blutgefäße abgedrückt und die Nährstoffversorgung des Nerven unterbrochen werden mit dem Effekt der Nervenschädigung.

Insbesondere bei Manschettenelektroden, die Nerven mit kleinen Durchmessern von einigen 10 µm bis wenige 100 µm umschließen sollen, sind die oben genannten Anforderungen in Kombination durch bestehende Technologien (Materialien und Designs) nicht befriedigend erfüllt, u.a. weil weiche Manschetten typischerweise von Hand hergestellt werden (und damit die erforderliche Genauigkeit für diese geometrische Größenordnung nicht ausreicht) oder mittels photolithographischer Methoden, wobei typischerweise sehr viel härtere Materialien (z.B. Polyimid) als Manschettenmaterial Anwendung finden.

Bekannt sind sogenannte Spiralmanschetten (Spiral Cuffs), die typischerweise mehr als eine Wicklung benötigen, um dicht gegenüber dem Körpergewebe abzuschließen. Da die wichtigen elektrischen Strukturen am Innenmantel der Spiralmanschette sitzen, muss die Spiralmanschette vor der Platzierung um den Nerv erst einmal komplett aufgerollt werden, was wiederum mechanischen Stress auf die delikaten eingebetteten Metallstrukturen ausübt. Wenn die gewünschten Durchmesser der Manschette sehr klein sind, z.B. kleiner als 1 mm, werden größere Rollkräfte zum Öffnen der Manschette benötigt. Dies widerspricht den Anforderungen an die mechanischen Eigenschaften der Manschette, die einen kleineren und somit weicheren Nerv umschließen müssen: Größere Rollkräfte erfordern ein dickeres Material, was wiederum die Biegesteifigkeit erhöht. Wenn das eingebettete Metall beim völligen Aufrollen der Manschette plastisch verformt wird, ist die Rückkehr der Manschette auf den Solldurchmesser nicht mehr ohne Weiteres möglich.

US 2006/0116739 A1 betrifft eine Elektrodenanordnung, bei welcher eine schlauchförmige Manschette mittels Vorsprüngen, die an dem einen Ende angeordnet sind, in Öffnungen, die am anderen Ende angeordnet sind, eingerastet wird. Die Aufgabe wird durch die Manschettenelektrode und das Verfahren zur Herstellung der Manschettenelektrode gemäß den unabhängigen Ansprüchen gelöst.

Bereitgestellt wird also eine implantierbare Manschettenelektrode, aufweisend:
eine flexible Manschette in Form eines Schlauches mit einem Längsschlitz, wobei der Längsschlitz eine erste Kante und eine zweite Kante an der Manschette definiert, wobei an der ersten Kante des Längsschlitzes eine erste Lippe angeordnet ist und an der zweiten Kante des Längsschlitzes eine zweite Lippe angeordnet ist, und wobei der Längsschlitz durch die erste Lippe und die zweite Lippe abdichtbar ist, indem die erste Lippe und die zweite Lippe zumindest teilweise auf dem Manschettenmantel der Manschette übereinander liegend verlaufen und die zweite Lippe die erste Lippe durch flächige Pressung auf dem Manschettenmantel in Position hält. Die zweite Lippe ist in Umfangsrichtung der Manschette relativ zur ersten Lippe und zum Manschettenmantel unter Erhaltung der Dichtung des Längsschlitzes beweglich.

Vorteilhafte Weiterbildungen sind wie folgt:
Die zweite Lippe kann bei Veränderung der Breite des Längsschlitzes unter Erhaltung der Abdichtung des Längsschlitzes beweglich sein.

Die Manschette kann am Längsschlitz durch Bewegen der zweiten Lippe unter elastischer Verformung der zweiten Lippe entgegen der Pressung und/oder des Manschettenmantels in Umfangsrichtung geöffnet werden.

Die Lippen können bei Veränderung des Abstands der Kanten zueinander in Umfangsrichtung der Manschette relativ zur Manschette unter Erhaltung der Abdichtung des Längsschlitzes beweglich sein.

Der Innendurchmesser der Manschette kann in Längsrichtung variieren, insbesondere zu den Enden hin zunehmen.

Auf der Innenwand der Manschette kann ein Elektrodenfeld angeordnet sein.

Das Elektrodenfeld kann zumindest zwei voneinander getrennte Sensorflächen umfassen, die längs einer Linie in Längsrichtung der Manschette angeordnet sind, wobei die zumindest zwei Sensorflächen über zumindest einen Leiterabschnitt miteinander in elektrischer Verbindung sind, wobei der zumindest eine Leiterabschnitt beim Öffnen und Schließen der Manschette elastisch verformbar ist.

Der zumindest eine Leiterabschnitt kann in der Innenwand angeordnet ist und in der Innenwand angeordnete mäanderförmige Muster umfassen.

Die Linie kann im Querschnitt der Manschettenelektrode diametral gegenüber dem Längsschlitz liegen und den Scheitelpunkt der Biegelinie im Querschnitt des Trägers umfassen.

Die Sensorflächenkönnen mit elektrischen Leitern gekoppelt sein, die nach außerhalb der Manschette herausgeführt sind.

Die Manschette kann ein Silikonschlauch sein, in welchem das Elektrodenfeld angeordnet ist, wobei das Elektrodenfeld in einen Träger aus Silikonmaterial eingebettet ist und wobei der Träger zwei Längsseiten aufweist, die aus dem Silikonschlauch herausgeführt sind und die Lippen bilden.

Über dem Längsschlitz kann eine Deckschicht angeordnet sein, welche zur Fixierung der Lippen und/oder zur Dichtung des Längsschlitzesbeiträgt.

Die Deckschicht kann Silikonmaterial umfassen.

Die erste Lippe kann über die zweite Lippe und über die Deckschicht hinausstehen.

Der Träger kann an den Längsseiten geringere Dicken aufweisen als in demjenigen Bereich, der im Inneren des Silikonschlauches angeordnet ist.

Der Träger kann eine sich von seiner Längsmittelachse in Richtung seiner Längsseiten verringernde Steifigkeit aufweisen.

Die Erfindung umfasst auch ein Verfahren zum Herstellen einer implantierbaren Manschettenelektrode, insbesondere gemäß einem der vorherigen Ansprüche, aufweisend:
Aufbringen einer ersten Schicht aus mit n-Heptan verdünntem Silikongummi auf einen Hilfsträger,
Aushärten der ersten Schicht,
Schneiden von Öffnungen in die erste Schicht,
Laminieren einer Metallfolie auf die erste Schicht,
Verringern der Dicke der Metallfolie an Stellen, die als Kontaktelektroden bestimmt sind,
Strukturieren der Metallfolie,
Aufbringen einer zweiten Schicht aus mit n-Heptan verdünntem Silikongummi,
Verbinden der der ersten Schicht aus Silikongummi und der zweiten Schicht aus Silikongummi durch Vulkanisation,
Freilegen der Stellen, die als Kontaktelektroden bestimmt sind,
Verringern der Dicke der Schicht aus Silikongummi an zwei gegenüberliegenden Rändern,
Entfernen des Hilfsträgers,
Anbringen von Drähten auf der Rückseite des Feldes,
Aufbringen einer dritten Schicht aus mit n-Heptan verdünntem Silikongummi auf die Rückseite des Feldes,
Umbiegen des Feldes über die zwei Ränder und Einbringen des umgebogenen Feldes in einen längsgeschlitzten Silikonschlauch, derart, dass die beiden Ränder sich außerhalb des Silikonschlauches befinden,
Aushärten der dritten Silikonschicht,
Anordnen der Ränder und Fixieren der Ränder mit einer Silikongummischicht, und
Aushärten der vierten Silikongummischicht.

Die Metallschicht kann an den Stellen, die Elektroden bilden, aufgeraut werden.

Das Metall kann mittels Laserstrahlen strukturiert werden.

Die Metallfolie in Querrichtung mäanderförmig strukturiert werden.

Silikon ist ein elastischer Werkstoff von besonderer Weichheit, mit hervorragender mechanischer und dielektrischer Langzeitstabilität in biologischer Umgebung und exzellenter biologischer Verträglichkeit. Silikon- und Elektrodenkontakte werden derart zu einer röhrenförmigen Manschette umgeformt, dass das Ergebnis den oben genannten Anforderungen entspricht. Besonders herauszuheben bei der hier vorgestellten Manschettenelektrodentechnologie ist der Verschlussmechanismus, der, anders als beim Stand der Technik, einen elektrischen Abdichtmechanismus der Manschette an der Manschettenaußenseite vorsieht. Eine solche Konstruktion kann durch Kombination weniger feinwerktechnischer Fertigungsschritte und hochpräziser Laser-Mikrostrukturierung produziert werden.

Der (selbstabdichtenden) Verschlussmechanismus ist an der Außenseite der Manschette angeordnet und bedarf keiner Dichtungslippe, die in den Innenraum der Manschette hineinragt.

Der Verschlussmechanismus ist derart gestaltet, dass beide Dichtungslippen leicht mit jeweils einer Pinzetten zu greifen sind. Durch Auseinanderziehen der Pinzette öffnet sich die Manschette.

Die elektrischen Kontakte innerhalb der Manschette sind so gestaltet, dass ein elastisches Öffnen der Manschette möglich ist ohne die Metallkontakte plastisch zu verformen.

Die Wand der Manschette kann zu den Enden in longitudinaler Richtung graduell dünner und damit die Steifigkeit geringer werden. Damit wird das Risiko der mechanischen Irritation des Nervs an den Enden der Manschette vermindert.

Für den Anwender ergeben sich folgende Vorteile.

Durch Maximierung der nutzbaren Fläche auf der Manschetteninnenwand kann eine bessere elektrische Kopplung (größere Kontaktflächen oder höhere Anzahl von kleinen Kontaktflächen) erzielt werden. Dies ist möglich durch die Verlegung des Verschlussmechanismus an die Manschettenaußenwand.

Auch bei Nervschwellungen erfährt der Nerv keine Kompression durch die Manschette, da Silikonlippen, die den Verschlussmechanismus an der Außenwand darstellen, zur Manschettendurchmesservergrößerung aneinander vorbei gleiten können, ohne die Verschlussqualität (elektr. Isolation) einzuschränken.

Vereinfachte Handhabung der Manschettenelektroden durch optische Kennzeichnung der äußersten Kante der Silikonlippen. Hier können die Lippen sehr einfach mit jeweils einer Pinzette oder Klemme angefasst und geöffnet werden, um den Nerv in die geöffnete Cuff einlegen zu können.

Hohe Zuverlässigkeit der Metallkontakte durch die Option, die Kontakte in mehrere, miteinander elektrisch verbundene Kontaktinseln aufzuteilen, so dass beim Öffnen der Manschette kein Metall plastisch verformt wird (was der Rückformung beim Schließen entgegenwirken würde), sondern eine scharnierartiges Aufklappen der Manschette möglich ist.

Durch graduelles Ausdünnen des Manschettenkörpers an den Enden wird die Steifigkeit verringert und das Risiko einer mechanischen Irritation des Nervs insbesondere an den Manschettenkanten minimiert.

Die Erfindung und Ausführungsbeispiele werden wird anhand der Zeichnung näher beschrieben. In der Zeichnung zeigen
- Fig. 1: eine perspektivische Ansicht der Manschettenelektrode in longitudinaler Richtung im Dichtungszustand,
- Fig. 2: eine Querschnittsansicht durch die Manschettenelektrode im Dichtungszustand,
- Fig. 3: eine Querschnittsansicht durch die Manschettenelektrode im Dichtungszustand, aber bei anschwellendem Nerv (der Längsschlitz ist etwas vergrößert),
- Fig. 4: eine Querschnittsansicht durch die Manschettenelektrode im geöffneten Zustand,
- Fig. 5: ein Elektrodenfeld in "abgewickelter" Ansicht, und

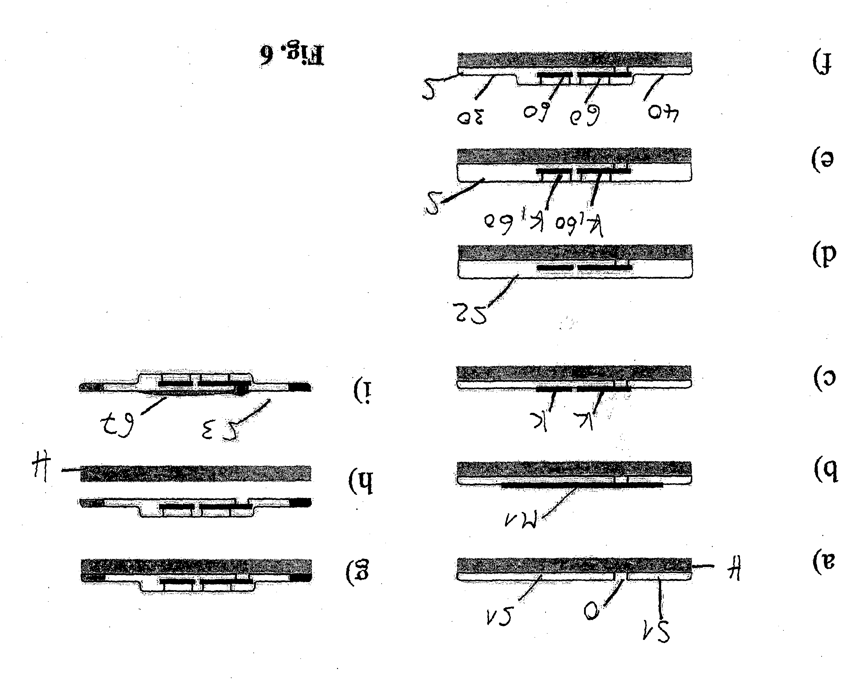

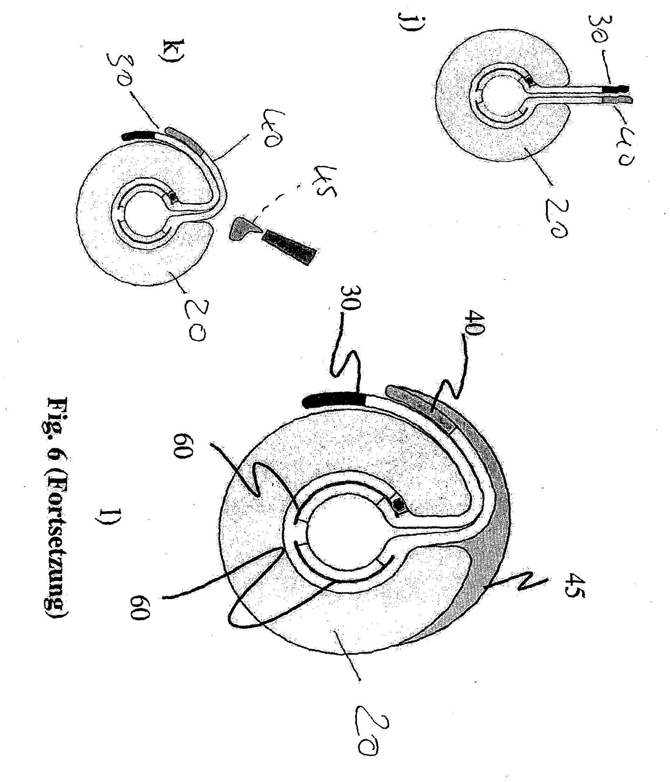

- Fig. 6: den Herstellungsprozess.

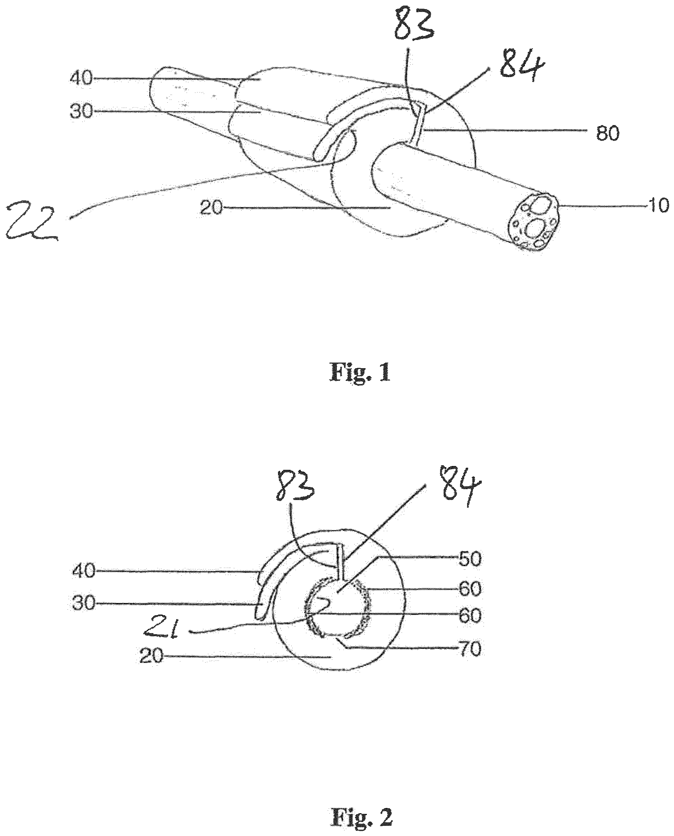

Fig. 1 zeigt eine perspektivische Ansicht der implantierbaren Manschettenelektrode in longitudinaler Richtung im Dichtungszustand. Manschette 20 umfasst die erste Lippe 30 und die zweite Lippe 40. Die Manschettenelektrode ist um den Nerv 10 herum gelegt. Die Manschettenelektrode ist den Nerv 10 herum anordenbar, d.h. wie ein Mantel um den Nerv 10 herum in longitudinaler Richtung des Nervs platzierbar. Die Manschettenelektrode umschließt also mit ihrer Manschette 20 den Nerv 10.

In der gesamten Beschreibung bedeutet "in Längsrichtung" (der Manschette) soviel wie "axial". Querschnitte sind senkrecht hierzu orientiert.

Fig. 2 zeigt eine Querschnittsansicht durch die Manschettenelektrode im Abdichtungszustand. Die Sensorfläche 60 ist so gestaltet, dass sie an mindestens einer Stelle 70 unterbrochen ist. Die Lippen 30 und 40 liegen beide außerhalb der Manschette 20 und erlauben dadurch eine optimale Nutzung der Manschetteninnenwand 21 für die Elektrodenkontakte 60.

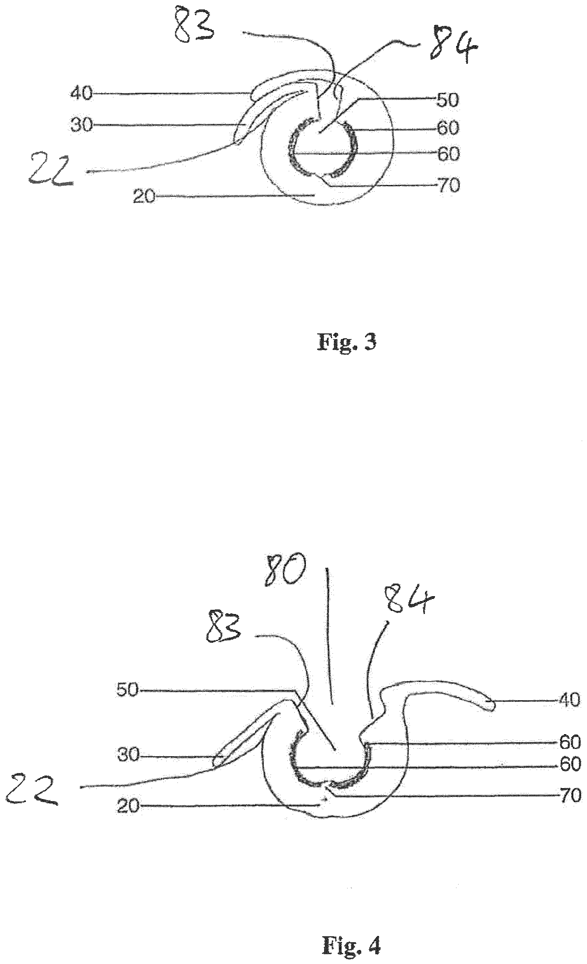

Fig. 3 zeigt eine Querschnittsansicht durch die Manschettenelektrode im Abdichtungszustand, aber bei anschwellendem Nerv (der Längsschlitz ist etwas vergrößert).

Fig. 4 zeigt eine Querschnittsansicht durch die Manschettenelektrode im geöffneten Zustand.

Die implantierbare Manschettenelektrode der Erfindung umfasst also eine flexible Manschette 20 in Form eines Schlauches mit einem Längsschlitz 80, wobei der Längsschlitz 80 eine erste Kante 83 und eine zweite Kante 84 an der Manschette 20 definiert, wobei an der ersten Kante 83 des Längsschlitzes 80 eine erste Lippe 30 angeordnet ist und an der zweiten Kante 84 des Längsschlitzes 80 eine zweite Lippe 40 angeordnet ist, und wobei der Längsschlitz 80 durch die erste Lippe 30 und die zweite Lippe 40 abdichtbar ist, indem die erste Lippen 30 und die zweite Lippe 40 zumindest teilweise auf dem Manschettenmantel 22 der Manschette 20 übereinander liegend verlaufen und die zweite Lippe 40 die erste Lippe 30 durch flächige Pressung auf dem Manschettenmantel 22 in Position hält.

Zumindest die zweite Lippe 40 ist in Umfangsrichtung der Manschette 20 relativ zur ersten Lippe 30 und zum Manschettenmantel unter Erhaltung der Abdichtung des Längsschlitzes 80 beweglich.

Die zweite Lippe 40 ist zusätzlich bei Veränderung der Breite des Längsschlitzes 80 unter Erhaltung der Abdichtung des Längsschlitzes beweglich.

Wie in Fig. 3 gezeigt ist, sind die Lippen 30, 40 bei Veränderung des Abstands der Kanten zueinander in Umfangsrichtung der Manschette 20 relativ zur Manschette 20 unter Erhaltung der Abdichtung des Längsschlitzes beweglich.

Wie in Fig. 4 gezeigt ist, kann die Manschette 20 am Längsschlitz durch Bewegen der zweiten Lippe 40 unter elastischer Verformung der zweiten Lippe 40 entgegen der Pressung und/oder des Manschettenmantels in Umfangsrichtung geöffnet werden.

Der Innendurchmesser der Manschette 20 kann in Längsrichtung variieren, z.B. zu den Enden hin zunehmen.

Auf der Innenwand 21 der Manschette ist ein Elektrodenfeld 60 angeordnet, wie in Fig. 3 und Fig. 4 gezeigt ist.

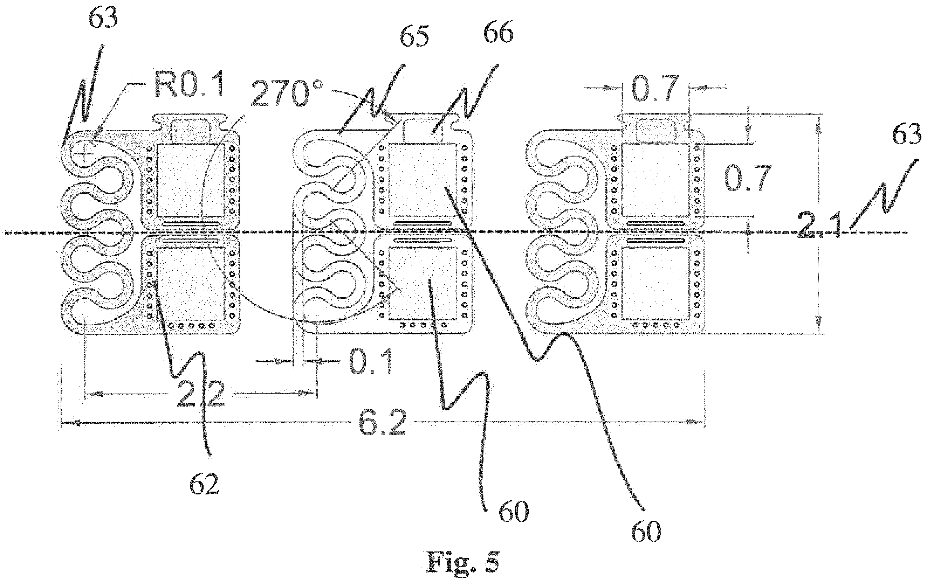

Fig. 5 zeigt ein Elektrodenfeld 60, 65 mit beispielhaften Bemaßungen. Das Elektrodenfeld 60, 65 umfasst zumindest zwei voneinander getrennte Sensorflächen 60, die längs einer Linie 70 in Längsrichtung der Manschette 20 angeordnet sind, wobei die zumindest zwei Sensorflächen 60 über zumindest einen Leiterabschnitt 65 miteinander in elektrischer Verbindung sind, wobei der zumindest eine Leiterabschnitt 65 beim Öffnen und Schließen der Manschette 20 elastisch verformbar ist.

Der zumindest eine Leiterabschnitt 65 ist in der Innenwand 21 angeordnet und umfasst in der Innenwand 21 angeordnete mäanderförmige Muster 63. Die mäanderförmigen Leiterabschnitte

Die Linie 70 liegt im Querschnitt der Manschettenelektrode diametral gegenüber dem Längsschlitz 80 und umfasst den Scheitelpunkt der Biegelinie im Querschnitt des Trägers 25, vgl. Fig. 2 bis 4.

Die Sensorflächen 60 sind mit elektrischen Leitern (nicht gezeigt) gekoppelt, die nach außerhalb der Manschette 20 herausgeführt sind. Hierzu umfasst das Elektrodenfeld 60, 65 Kontaktpads 66, an denen die Leiter angeschlossen sind.

Speziell kann die Manschette 20 ein Silikonschlauch sein, in welchem das Elektrodenfeld 60, 65 angeordnet ist, wobei das Elektrodenfeld 60, 65 seinerseits in einen Träger 25 ebenfalls aus Silikonmaterial eingebettet ist und wobei der Träger 25 zwei Längsseiten aufweist, die aus dem Silikonschlauch herausgeführt sind und die erste Lippe 30 und die zweite Lippe 40 bilden.

Über dem Längsschlitz 80 kann eine Deckschicht 45 angeordnet sein, welche zur Fixierung der Lippen 30, 40 und/oder zur Dichtung des Längsschlitzes 80 beiträgt. Die Deckschicht 45 kann ebenfalls Silikonmaterial umfassen. Die erste Lippe 30 kann über die zweite Lippe 40 und über die Deckschicht 45 hinausstehen.

Der Träger 25 kann an den Längsseiten geringere Dicken aufweisen als in demjenigen Bereich, der im Inneren des Silikonschlauches angeordnet ist. Der Träger 25 kann eine sich von seiner Längsmittelachse in Richtung seiner Längsseiten verringernde Steifigkeit aufweisen.

Das erfindungsgemäße Herstellungsverfahren ermöglicht die Formgebung der Manschettenelektrode durch eine vorgeformte halb-zylindrische Form eines gewünschten Durchmessers und mit gewünschten mechanischen Eigenschaften der Manschettenelektrode.

Durch die Prozessabfolge kann mit einer veränderten Dicke des Trägersilikons (Laser-Elektrodenarray) der Innendurchmesser der 3-D Struktur definiert werden. Eine lokale Strukturierung des Trägersilikons erlaubt eine lokale Dickenanpassung der Trägerstruktur, um unabhängig vom Durchmesser den Lippenverschlussmechanismus aufrecht zu erhalten.

Die Manschette besitzt einen selbst schließenden Mechanismus, welcher die Manschette unabhängig von der Öffnungsbelastung auf ihren Solldurchmesser zurück bringt und den Längsschlitz dichtend verschließt.

Durch die Lage und Architektur des eingebetteten Metalls übt das Metall keine Kraft auf die Manschette aus und beeinträchtigt somit die Sollform der Manschette nicht.

Die Verschlusslippen haben eine vom Manschettendurchmesser unabhängige Verschlusskraft, die bei eventuellen Schwellungen der Nerven einen dichten Verschluss der Manschette garantiert. Diese Verschlusslippen dienen gleichzeitig als Greifstrukturen, die das Öffnen der Manschette ohne Krafteinwirkung auf die kritischen Metallstrukturen ermöglichen.

Eine graduelle Änderung der Manschettensteifigkeit zum Rand hin ermöglicht den Schutz der Nerven und verhindert das scharfe Abknicken der Nerven über eine Kante an den Enden der Manschette.

Die gezielte Laserstrukturierung erlaubt eine lokale Veränderung der mechanischen Eigenschaften der metalltragenden Silikonstruktur. Somit lässt die eigene Kraft der geformten Trägerfolie lokal anpassen und eine Umformung der planaren Struktur wird ermöglicht.

Die gezielte Laserstrukturierung erlaubt eine lokale Veränderung der geometrischen Eigenschaften der metalltragenden Silikonstruktur. Somit lässt der Innendurchmesser der Manschette 20 mittels der Trägerfolie lokal anpassen ohne die Eigenschaften des Verschlussmechanismus zu verändern.

Eine graduelle Anpassung der Manschettensteifigkeit zum Rand hin ermöglicht den Schutz der Nerven und verhindert das scharfe Abknicken der Nerven über eine Kante an den Enden der Manschette.

Das Herstellungsverfahren basiert auf einem planaren Elektrodenfeld, welches auf Strukturieren von Metallfolie mittels Laserstrahlen und Silikongummi basiert. Das Elektrodenfeld wird in einen Silikonschlauch eingefügt für anschließendes 3-D-Formen. Das Elektrodenfeld wird aus Silikongummi für medizinische Anwendungen (Medical Grade PDMS, MED 1000) und Platinfolie mit einer Ausgangsdicke von 12,5 µm (99,5 % Pt) hergestellt. Anstelle der Pt-Folie kann auch Folie aus PtIr verwendet werden (z.B. 90 % Pt, 10% Ir). Zum Strukturieren des Elektrodenfeldes kann ein Nd:YVO₄-Pikosekunden-Laser mit einer dritten harmonischen Oberwelle von 355 nm verwendet werden.

Als Hilfsträger während des Herstellungsprozesses wird ein 2" mal 2" Aluminiumsubstrat verwendet, das mit einem selbstklebenden Film versehen ist, der zu Ende des Strukturierungsvorganges abgelöst wird.

Das PDMS wird in 1:1 mit n-Heptan verdünnt, um seine Viskosität zu verringern und ein Aufschleudern (Spin Coating) zu ermöglichen.

Das Verfahren zum Herstellen einer implantierbaren Manschettenelektrode weist die folgenden Schritte auf:
a) Aufbringen einer ersten Schicht aus mit n-Heptan verdünntem Silikongummi (PDMS) auf einem Hilfsträger H, diese Schicht ist vorzugsweise 100 µm stark und wird vorzugsweise auf den mit dem selbstklebendem Film versehenen Hilfsträger aufgeschleudert;
b) Aushärten der ersten Schicht S1;
c) Schneiden von Öffnungen O in die erste Schicht S1 diese Öffnungen bilden die Öffnungen in den Verbindungspads, an welchen die Drähte angeschlossen werden (Fig. 1a);
d) Laminieren einer Metallfolie M1 auf die erste Schicht; die Metallfolie ist vorzugsweise gereinigte Pt-Folie (Fig. 1b);
f) Verringern der Dicke der Metallfolie an Stellen K, die als Kontaktelektroden 60 bestimmt sind;
f) Strukturieren der Metallfolie (Fig. 1c); das überschüssige Matetrial wird von der Oberfläche entfernt;
g) Aufbringen einer zweiten Schicht S2 aus mit n-Heptan verdünntem Silikongummi, um die Metallfolie einzubetten, vorzugsweise verdünntes PDMS einer Stärke von 100 µm (Fig. 1d); der Innendurchmesser der Manschettenelektrode kann durch Variieren der Stärke dieser Schicht verändert werden;
h) Verbinden der der ersten Schicht S1 aus Silikongummi und der zweiten Schicht S2 aus Silikongummi durch Vulkanisation zu einer Silikongummischicht S,
i) Freilegen der Stellen, die als Kontaktelektroden 60 bestimmt sind, vorzugsweise mittels des Lasers (Fig. 1e); mittels des Lasers können auch die ebenen Stellen der Kontaktelektroden aufgeraut werden, so dass eine höhere Ladungsinjektionsfähigkeit entsteht;
j) Verringern der Stärke der Silikongummischicht an zwei gegenüberliegenden Längsrändern (Fig. 1f); vorzugsweise auf 75 µm; die Bereiche verringerter Stärke werden nach außerhalb des Manschetteninneren geführt und bilden die Lippen 30, 40 der Manschettenelektrode; durch Reduzieren der Stärke der Silikongummischicht erhält man eine bessere Formbarkeit beim letzten Gestaltungsschritt (Fig. 1k); in diesem Schritt kann auch die äußere Form der Silikongummischicht zurechtgeschniten werden;
l) Nach Trocknen werden die beiden Lippen 30, 40 eingefärbt, vorzugsweise mit zwei optisch unterscheidbaren Farben; dies ermögliche eine bessere Unterscheidbarkeit der Lippen beim Implantieren der Manschettenelektrode (Fig. 1g);
m) Entfernen des Hilfsträgers H (Fig. 1h);
n) Anbringen von Drähten 67 auf der Rückseite des Feldes, vorzugsweise Löten von Drähten mit einem Durchmesser von 70 bis 80 µm;
o) Aufbringen einer dritten Schicht S3 aus mit n-Heptan verdünntem Silikongummi auf die Rückseite des Feldes (Fig. 1i).
   Das Elektrodenfeld ist nun fertig zum Einführen in den Silikonschlauch. Beim Silikonschlauch handelt es sich vorzugsweise um einen USP-Class VI Silikonschlauch mit einem für den Außendurchmesser des Nervs passenden Innendurchmesser und einer Wandstärke von 300 bis 500 µm. Der Silikonschlauch wird auch die gewünschte Länge zurechtgeschnitten und mit einem geradlinigen Längsschlitz 80 parallel zur Längsachse des Silikonschlauch versehen.
p) Umbiegen des Feldes über die zwei Ränder, derart, dass sich die beiden Lippen 30, 40 berühren. Die Querschnitts-Biegelinie des Feldes ohne die Lippen 30, 40 beschreibt eine Kreislinie; Einbringen des umgebogenen Feldes in den längsgeschlitzten Silikonschlauch, derart, dass die beiden Lippen durch den Längsschlitz 80 hindurchtreten und sich außerhalb des Silikonschlauches befinden (Fig. 1j). Das Einbringen des Elektrodenfeldes 60, 65 in den Silikonschlauch kann mittels einer Kanüle erfolgen.
q) Aushärten der dritten Silikongummischicht; hierbei kann die Manschettenelektrode in einer Halfpipe-Teflon-Form (Durchmesser entspricht Außendurchmesser des Silikonschlauchs) stabilisiert werden.
r) Umfalten der Lippen 30, 40 auf den Mantel des Silikonschlauches und Fixieren der äußeren Lippe 40 mit einer Verstärkungsschicht 45 aus verdünntem Silikongummi, um die Lippe 40 in ihrer Position zu halten. Diese Verstärkungsschicht bildet zusammen mit den beiden Lippen ein selbstschließendes System (Fig. lk).
s) Aushärten der Verstärkungsschicht. Es entsteht die Manschettenelektrode gemäß Fig. 1L).

Die beiden Lippen 30, 40 stellen einen Verschlussmechanismus bereit, der nicht zur Kompression des Nervs 10 beiträgt. Die obenliegende zweite Lippe 40 wird neu geformt, wenn sie nach dem Umbiegen mit dem n-Heptan-verdünnten PDMS behandelt wird, wohingegen die untere Lippe, also diejenige, welche den Silikonschlauch direkt berührt, nur durch die Lippe 1 in Position gehalten wird. Die Lippe 2 wirkt also gegen die Lippe 1 und schafft somit einen guten Kontakt zwischen den Lippen im Längsschlitz. Dies schafft überdies den selbstschließenden Verschlussmechanismus.

Die Lippen sind vorzugsweise metallfrei, um die Dichtwirkung an dem Schlitz nach der Implantation zu verbessern.

Beim Aufbiegen der Manschettenelektrode vor dem Implantieren um den Nerv herum erfährt die Manschettenelektrode große Druck- oder Zugbelastungen über ihrem Querschnitt.

Wegen der wesentlich geringeren Dehnbarkeit von Metall im Vergleich zu Silikongummi droht eine Ablösung (Delamination) des Silikongummis vom Metall.

Andererseits drohen Brüche dort, wo das Metall beim Aufbiegen die höchste Belastung erfährt. Diese ist die Scheitellinie des gebogenen Metalls, die in Längsrichtung entlang der Symmetrielinie des Trägers verläuft.

Um ein einen Bruch dort zu vermeiden, wird das Metall entlang der Scheitellinie (Symmetrielinie) unterbrochen. Die Elektrodenfläche ist dort also zweigeteilt. Um eine elektrische Verbindung beider Elektrodenflächen herzustellen, sind die beiden Elektrodenflächen über eine Metallbahn miteinander verbunden. Diese Metallbahn verläuft mäanderförmig in Umfangsrichtung des gebogenen Elektrodenfeldes, also quer Symmetrielinie des Trägers. Vorzugsweise gibt es mehr als einen Mäanderabschnitt und die Mäanderabschnitte beschreiben vorzugsweise Dreiviertelkreise. Die Metallbahn hat vorzugsweise eine Breite von 100 µm und die Mäanderabschnitte verlaufen in ¾-Kreis-Schleifen. Die Schleifen haben vorzugsweise einen Durchmesser von 0,1 mm.

Es können mehrere Elektroden in Längsrichtung gebildet sein, z.B. zwei oder drei Elektroden.

Eine typische Länge der Manschettenelektrode beträgt 6,2 mm, der Außendurchmesser kann 2,1 mm betragen.

## Patentansprüche

1. Implantierbare Manschettenelektrode, aufweisend:
eine flexible Manschette (20) in Form eines Schlauches mit einem Längsschlitz (80), wobei der Längsschlitz (80) eine erste Kante (83) und eine zweite Kante (84) an der Manschette (20) definiert, wobei an der ersten Kante (83) des Längsschlitzes (80) eine erste Lippe (30) angeordnet ist und an der zweiten Kante (84) des Längsschlitzes (80) eine zweite Lippe (40) angeordnet ist, und wobei der Längsschlitz (80) durch die erste Lippe (30) und die zweite Lippe (40) abdichtbar ist, indem die erste Lippe (30) und die zweite Lippe (40) zumindest teilweise auf dem Manschettenmantel (22) der Manschette (20) übereinander liegend verlaufen,
**dadurch gekennzeichnet, dass**
die zweite Lippe (40) die erste Lippe (30) durch flächige Pressung auf dem Manschettenmantel (22) in Position hält und zumindest die zweite Lippe (40) in Umfangsrichtung der Manschette (20) relativ zur ersten Lippe (30) und zum Manschettenmantel (22) unter Erhaltung der Dichtung des Längsschlitzes (80) beweglich ist.

2. Manschettenelektrode gemäß Anspruch 1, wobei zumindest die zweite Lippe (40) bei Veränderung der Breite des Längsschlitzes (80) unter Erhaltung der Abdichtung des Längsschlitzes beweglich ist.

3. Manschettenelektrode gemäß einem der vorherigen Ansprüche, wobei die Manschette am Längsschlitz durch Bewegen der zweiten Lippe (40) unter elastischer Verformung der zweiten Lippe (40) entgegen der Pressung und/oder des Manschettenmantels (22) in Umfangsrichtung geöffnet werden kann.

4. Manschettenelektrode gemäß einem der vorherigen Ansprüche, wobei die Lippen (30, 40) bei Veränderung des Abstands der Kanten zueinander in Umfangsrichtung der Manschette (20) relativ zur Manschette (20) unter Erhaltung der Abdichtung des Längsschlitzes (80) beweglich sind.

5. Manschettenelektrode gemäß einem der vorherigen Ansprüche, wobei auf der Innenwand (21) der Manschette (20) ein Elektrodenfeld (60, 65) angeordnet ist.

6. Manschettenelektrode gemäß dem vorherigen Anspruch, wobei das Elektrodenfeld (60, 65) zumindest zwei voneinander getrennte Sensorflächen (60) umfasst, die längs einer Linie (70) in Längsrichtung der Manschette (20) angeordnet sind, wobei die zumindest zwei Sensorflächen über zumindest einen Leiterabschnitt (65) miteinander in elektrischer Verbindung sind, wobei der zumindest eine Leiterabschnitt (65) beim Öffnen und Schließen der Manschette (20) elastisch verformbar ist.

7. Manschettenelektrode gemäß dem vorherigen Anspruch, wobei der zumindest eine Leiterabschnitt (65) in der Innenwand (21) angeordnet ist und in der Innenwand (21) angeordnete mäanderförmige Muster (63) umfasst.

8. Manschettenelektrode gemäß einem der Ansprüche 5 bis 7, wobei die Manschette (20) ein Silikonschlauch ist, in welchem das Elektrodenfeld (60, 65) angeordnet ist, wobei das Elektrodenfeld (60, 65) in einen Träger (25) aus Silikonmaterial eingebettet ist und wobei der Träger (25) zwei Längsseiten aufweist, die aus dem Silikonschlauch herausgeführt sind und die Lippen (30, 40) bilden.

9. Manschettenelektrode gemäß einem der vorherigen Ansprüche, wobei über dem Längsschlitz (80) eine Deckschicht (45) angeordnet ist, welche zur Fixierung der Lippen (30, 40) und/oder zur Dichtung des Längsschlitzes (80) beiträgt.

10. Verfahren zum Herstellen einer implantierbaren Manschettenelektrode, gemäß Anspruch 5, aufweisend:
Aufbringen einer ersten Schicht aus mit n-Heptan verdünntem Silikongummi auf einen Hilfsträger,
Aushärten der ersten Schicht,
Schneiden von Öffnungen in die erste Schicht,
Laminieren einer Metallfolie auf die erste Schicht,
Verringern der Dicke der Metallfolie an Stellen, die als Kontaktelektroden bestimmt sind,
Strukturieren der Metallfolie,
Aufbringen einer zweiten Schicht aus mit n-Heptan verdünntem Silikongummi,
Verbinden der ersten Schicht aus Silikongummi und der zweiten Schicht aus Silikongummi durch Vulkanisation,
Freilegen der Stellen, die als Kontaktelektroden bestimmt sind,
Verringern der Dicke der Schicht aus Silikongummi an zwei gegenüberliegenden Rändern,
Entfernen des Hilfsträgers,
Anbringen von Drähten auf der Rückseite des Feldes,
Aufbringen einer dritten Schicht aus mit n-Heptan verdünntem Silikongummi auf die Rückseite des Feldes,
Umbiegen des Feldes über die zwei Ränder und Einbringen des umgebogenen Feldes in einen längsgeschlitzten Silikonschlauch, derart, dass die beiden Ränder sich außerhalb des Silikonschlauches befinden, Aushärten der dritten Silikonschicht,
Anordnen der Ränder und Fixieren der Ränder mit einer Silikongummischicht, und
Aushärten der vierten Silikongummischicht.

## Claims

1. Implantable cuff electrode, comprising:
a flexible cuff (20) in the form of a tube having a longitudinal slot (80), the longitudinal slot (80) defining a first edge (83) and a second edge (84) on the cuff (20), a first lip (30) being arranged on the first edge (83) of the longitudinal slot (80) and a second lip (40) being arranged on the second edge (84) of the longitudinal slot (80), and it being possible to seal the longitudinal slot (80) by means of the first lip (30) and the second lip (40) by the first lip (30) and the second lip (40) at least partly extending on the cuff jacket (22) of the cuff (20) so as to lie one on top of the other,
**characterized in that**
the second lip (40) holds the first lip (30) in position by surface pressure on the cuff jacket (22) and at least the second lip (40) is movable in the circumferential direction of the cuff (20) relative to the first lip (30) and to the cuff jacket (22) while maintaining the sealing of the longitudinal slot (80).

2. Cuff electrode according to claim 1, wherein, when changing the width of the longitudinal slot (80), at least the second lip (40) is movable while maintaining the sealing of the longitudinal slot.

3. Cuff electrode according to either of the preceding claims, wherein the cuff can be opened at the longitudinal slot by moving the second lip (40) against the pressure and/or the cuff jacket (22) in the circumferential direction, with elastic deformation of the second lip (40).

4. Cuff electrode according to any of the preceding claims, wherein, when changing the distance between the edges in the circumferential direction of the cuff (20), the lips (30, 40) are movable relative to the cuff (20) while maintaining the sealing of the longitudinal slot (80).

5. Cuff electrode according to any of the preceding claims, wherein an electrode field (60, 65) is arranged on the inner wall (21) of the cuff (20).

6. Cuff electrode according to the preceding claim, wherein the electrode field (60, 65) comprises at least two separate sensor surfaces (60) which are arranged along a line (70) in the longitudinal direction of the cuff (20), the at least two sensor surfaces being electrically interconnected via at least one conductor portion (65), the at least one conductor portion (65) being elastically deformable when the cuff (20) is opened and closed.

7. Cuff electrode according to the preceding claim, wherein the at least one conductor portion (65) is arranged in the inner wall (21) and comprises meandering patterns (63) arranged in the inner wall (21).

8. Cuff electrode according to any of claims 5 to 7, wherein the cuff (20) is a silicone tube in which the electrode field (60, 65) is arranged, the electrode field (60, 65) being embedded in a carrier (25) made of silicone material and the carrier (25) having two long sides which are led out of the silicone tube and form the lips (30, 40).

9. Cuff electrode according to any of the preceding claims, wherein a cover layer (45) is arranged over the longitudinal slot (80) and contributes to the securing of the lips (30, 40) and/or to the sealing of the longitudinal slot (80).

10. Method for producing an implantable cuff electrode according to claim 5, comprising:
applying a first layer of silicone rubber diluted with n-heptane to an auxiliary carrier,
curing the first layer,
cutting openings in the first layer,
laminating a metal foil to the first layer,
reducing the thickness of the metal foil at points which are intended as contact electrodes,
structuring the metal foil,
applying a second layer of silicone rubber diluted with n-heptane,
connecting the first layer of silicone rubber and the second layer of silicone rubber by vulcanization,
exposing the points which are intended as contact electrodes,
reducing the thickness of the layer of silicone rubber at two opposite edges,
removing the auxiliary carrier,
attaching wires to the back of the field,
applying a third layer of silicone rubber diluted with n-heptane to the back of the field,
bending the field over the two edges and inserting the bent field into a longitudinally slotted silicone tube such that the two edges are outside the silicone tube,
curing the third silicone layer,
arranging the edges and securing the edges to a silicone rubber layer, and
curing the fourth silicone rubber layer.

## Revendications

1. Électrode à gaine implantable comportant :
une gaine souple (20) en forme de tuyau présentant une fente longitudinale (80), la fente longitudinale (80) définissant une première arête (83) et une seconde arête (84) sur la gaine (20), une première lèvre (30) étant disposée sur la première arête (83) de la fente longitudinale (80) et une seconde lèvre (40) étant disposée sur la seconde arête (84) de la fente longitudinale (80), et la fente longitudinale (80) pouvant être fermée de manière étanche par la première lèvre (30) et la seconde lèvre (40), du fait que la première lèvre (30) et la seconde lèvre (40) s'étendent l'une sur l'autre au moins partiellement sur l'enveloppe (22) de la gaine (20),
**caractérisée en ce que**
la seconde lèvre (40) maintient la première lèvre (30) en position par pression à plat sur l'enveloppe (22) de la gaine, et **en ce qu'**au moins la seconde lèvre (40) est mobile dans la direction circonférentielle de la gaine (20) par rapport à la première lèvre (30) et à l'enveloppe (22) de la gaine tout en maintenant l'étanchéité de la fente longitudinale (80).

2. Électrode à gaine selon la revendication 1, dans laquelle au moins la seconde lèvre (40) est mobile lorsque la largeur de la fente longitudinale (80) est modifiée tout en maintenant l'étanchéité de la fente longitudinale.

3. Électrode à gaine selon l'une des revendications précédentes, dans laquelle la gaine peut être ouverte au niveau de la fente longitudinale par déplacement de la seconde lèvre (40) en déformant élastiquement la seconde lèvre (40) contre la pression et/ou l'enveloppe (22) de la gaine dans la direction circonférentielle.

4. Électrode à gaine selon l'une des revendications précédentes, dans laquelle, lorsque la distance entre les arêtes est modifiée, les lèvres (30, 40) sont mobiles par rapport à la gaine (20) dans la direction circonférentielle de la gaine (20) tout en maintenant l'étanchéité de la fente longitudinale (80).

5. Électrode à gaine selon l'une des revendications précédentes, dans laquelle un champ d'électrode (60, 65) est disposé sur la paroi interne (21) de la gaine (20).

6. Électrode à gaine selon la revendication précédente, dans laquelle le champ d'électrode (60, 65) comprend au moins deux surfaces de capteur (60) séparées l'une de l'autre et disposées le long d'une ligne (70) dans la direction longitudinale de la gaine (20), les au moins deux surfaces de capteur étant en connexion électrique l'une avec l'autre par l'intermédiaire d'au moins une section conductrice (65), l'au moins une section conductrice (65) pouvant être déformée élastiquement lorsque la gaine (20) est ouverte et fermée.

7. Électrode à gaine selon la revendication précédente, dans laquelle l'au moins une section conductrice (65) est disposée dans la paroi interne (21) et comprend des motifs en méandres (63) disposés dans la paroi interne (21).

8. Électrode à gaine selon l'une des revendications 5 à 7, la gaine (20) étant un tuyau de silicone dans lequel le champ d'électrode (60, 65) est disposé, le champ d'électrode (60, 65) étant noyé dans un support (25) en matériau de silicone, et le support (25) comportant deux côtés longitudinaux qui sortent du tuyau de silicone et forment les lèvres (30, 40).

9. Électrode à gaine selon l'une des revendications précédentes, dans laquelle une couche de recouvrement (45) est disposée au-dessus de la fente longitudinale (80), laquelle couche de recouvrement contribue à la fixation des lèvres (30, 40) et/ou à l'étanchéité de la fente longitudinale (80).

10. Procédé de fabrication d'une électrode à gaine implantable selon la revendication 5, comportant :
l'application, sur un support auxiliaire, d'une première couche constituée de caoutchouc de silicone dilué avec du n-heptane,
le durcissement de la première couche,
le découpage d'ouvertures dans la première couche,
le laminage d'une feuille métallique sur la première couche,
la réduction de l'épaisseur de la feuille métallique aux emplacements prévus comme électrodes de contact,
la structuration de la feuille métallique,
l'application d'une deuxième couche constituée de caoutchouc de silicone dilué avec du n-heptane,
la liaison de la première couche constituée de caoutchouc de silicone et de la deuxième couche constituée de caoutchouc de silicone par vulcanisation,
la mise à nu des emplacements prévus comme électrodes de contact,
la réduction de l'épaisseur de la couche constituée de caoutchouc de silicone sur deux bords opposés,
l'élimination du support auxiliaire,
la fixation des fils au dos du champ,
l'application d'une troisième couche constituée de caoutchouc de silicone dilué avec du n-heptane au dos du champ,
le pliage du champ sur les deux bords et
l'insertion du champ plié dans un tuyau de silicone fendu longitudinalement de telle manière que les deux bords se trouvent à l'extérieur du tuyau de silicone,
le durcissement de la troisième couche de silicone,
la disposition des bords et la fixation des bords à l'aide d'une couche de caoutchouc de silicone, et
le durcissement de la quatrième couche de caoutchouc de silicone.
